Europäisches Patentamt
European Patent Office
Office européen des brevets

(19)

(11) Veröffentlichungsnummer: **0 332 963 A1**

(12)

# EUROPÄISCHE PATENTANMELDUNG

(21) Anmeldenummer: **89103864.8**

(22) Anmeldetag: **06.03.89**

(51) Int. Cl.[4]: **C07D 239/47 , A01N 43/54**

(30) Priorität: **16.03.88 DE 3808739**

(43) Veröffentlichungstag der Anmeldung:
**20.09.89 Patentblatt 89/38**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Müller, Klaus-Helmut, Dr.**
**Bockhackstrasse 55**
**D-4000 Düsseldorf 13(DE)**
Erfinder: **Kluth, Joachim, Dr.**
**Kurt-Schumacher-Strasse 9**
**D-4018 Langenfeld(DE)**
Erfinder: **Tietjen, Klaus-Günther, Dr.**
**Am Alten Broich 64a**
**D-4018 Langenfeld(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Harry, Dr.**
**Unterdorfstrasse 6 A**
**D-4000 Düsseldorf 31(DE)**

(54) **Pyrimidin-Derivate.**

(57) Die Erfindung betrifft neue Pyrimidin-Derivate der allgemeinen Formel (I)

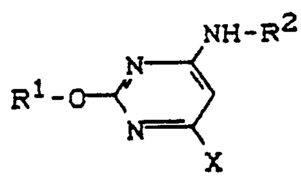

(I)

in welcher
$R^1$ für gegebenenfalls substituiertes Alkyl steht,
$R^2$ für einen gegebenenfalls substituierten Rest aus der Reihe Alkyl, Cycloalkyl, Cycloalkylalkyl, Aryl, Aralkyl, Heteroaryl und Heteroarylalkyl steht, und
X für Halogen, Amino, Cyanamino oder für einen gegebenenfalls substituierten Rest aus der Reihe Alkoxy, Alkylthio, Alkylamino und Dialkylamino steht,
Verfahren zu ihrer Herstellung und ihre Verwendung als Herbizide.

EP 0 332 963 A1

## Pyrimidin-Derivate

Die Erfindung betrifft neue Pyrimidin-Derivate, Verfahren zu ihrer Herstellung und ihre Verwendung als Herbizide.

Es ist bekannt, daß bestimmte Pyrimidin-Derivate, wie z. B. 2-Amino-4-chlor-6-isopropylamino-pyrimidin, herbizide Eigenschaften aufweisen (vgl. DE-OS 20 06 145). Die herbizide Wirkung der bekannten Pyrimidin-Derivate ist jedoch bei niedrigen Aufwandmengen unbefriedigend.

Es wurden nun die neuen Pyrimidin-Derivate der allgemeinen Formel (I)

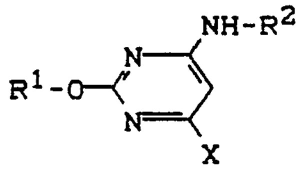

(I)

in welcher
$R^1$ für gegebenenfalls substituiertes Alkyl steht,
$R^2$ für einen gegebenenfalls substituierten Rest aus der Reihe Alkyl, Cycloalkyl, Cycloalkylalkyl, Aryl, Aralkyl, Heteroaryl und Heteroarylalkyl steht, und
X für Halogen, Amino, Cyanamino oder für einen gegebenenfalls substituierten Rest aus der Reihe Alkoxy, Alkylthio, Alkylamino und Dialkylamino steht,
gefunden.

Weiter wurde gefunden, daß man die neuen Pyrimidin-Derivate der allgemeinen Formel (I) erhält, wenn man

(a) 2-Alkylsulfonyl-pyrimidin-Derivate der allgemeinen Formel (II)

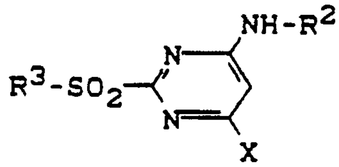

(II)

in welcher
$R^2$ und X die oben angegebenen Bedeutungen haben und
$R^3$ für gegebenenfalls substituiertes Alkyl oder Benzyl steht,
mit Metall-Alkoholaten der allgemeinen Formel (III)
$R^1$ - O - M (III)
in welcher
$R^1$ die oben angegebene Bedeutung hat und
M für ein Metalläquivalent steht,
gegebenenfalls in Gegenwart eines Verdünnungsmittels umsetzt, oder

(b) 4-Halogen-pyrimidin-Derivate der allgemeinen Formel (Ia)

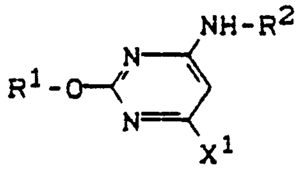

(Ia)

in welcher
$R^1$ und $R^2$ die oben angegebenen Bedeutungen haben und
$X^1$ für Halogen steht,
mit Verbindungen der allgemeinen Formel (IV)
$M^1$ - X (IV)

in welcher
X die oben angegebene Bedeutung hat und
$M^1$ für Wasserstoff oder ein Metalläquivalent steht,
gegebenenfalls in Gegenwart eines Säureakzeptors und gegebenenfalls in Gegenwart eines Verdünnungsmittels umsetzt, oder

(c) 4-Halogen-pyrimidin-Derivate der allgemeinen Formel (V)

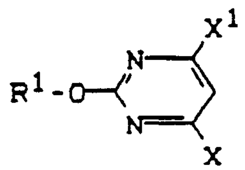

(V)

in welcher
$R^1$, X und $X^1$ die oben angegebenen Bedeutungen haben,
mit Aminoverbindungen der allgemeinen Formel (VI)
$H_2N - R^2$ (VI)
in welcher
$R^2$ die oben angegebene Bedeutung hat,
gegebenenfalls in Gegenwart eines Säureakzeptors und gegebenenfalls in Gegenwart eines Verdünnungsmittels umsetzt.

Die neuen Pyrimidin-Derivate der allgemeinen Formel (I) zeichnen sich durch starke herbizide Wirksamkeit aus.

Überraschenderweise zeigen die erfindungsgemäßen Verbindungen der allgemeinen Formel (I) bei guter Selektivität in Kulturpflanzen erheblich stärkere Wirkung gegen Unkräuter als nach Struktur und Wirkprofil vergleichbare bekannte Verbindungen.

Gegenstand der Erfindung sind vorzugsweise Verbindungen der Formel (I), in welcher
$R^1$ für gegebenenfalls durch Fluor, Chlor, Brom, $C_1$-$C_4$-Alkoxy (welches gegebenenfalls durch Fluor und/oder Chlor substituiert ist), $C_1$-$C_4$-Alkoxy-$C_1$-$C_4$-alkoxy, $C_1$-$C_4$-Alkylthio, $C_1$-$C_4$-Alkylamino oder Di-($C_1$-$C_4$-alkyl)-amino substituiertes $C_1$-$C_6$-Alkyl steht,
$R^2$ für gegebenenfalls durch Fluor, Chlor, $C_1$-$C_4$-Alkoxy, $C_1$-$C_4$-Alkylthio, $C_1$-$C_4$-Alkylamino oder Di-($C_1$-$C_4$-alkyl)-amino substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl oder $C_3$-$C_6$-Cycloalkyl-$C_1$-$C_3$-alkyl, oder für eine gegebenenfalls durch Fluor, Chlor, Brom, Iod, Cyano, Nitro, $C_1$-$C_4$-Alkyl (welches gegebenenfalls durch Fluor und/oder Chlor substituiert ist), $C_1$-$C_4$-Alkoxy oder $C_1$-$C_2$-Alkylendioxy (welche gegebenenfalls durch Fluor und/oder Chlor substituiert sind), $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), Di-($C_1$-$C_2$-alkyl)-amino und/oder durch $C_1$-$C_4$-Alkoxy-carbonyl substituierte Gruppierung aus der Reihe Phenyl, Naphthyl, Phenyl-$C_1$-$C_4$-alkyl, Naphthyl-$C_1$-$C_4$-alkyl, Pyridyl, Pyridyl-$C_1$-$C_4$-alkyl, Chinolinyl, Chinolinyl-$C_1$-$C_4$-alkyl, Isochinolinyl, Isochinolinyl-$C_1$-$C_4$-alkyl, Pyrimidinyl, Pyrimidinyl-$C_1$-$C_4$-alkyl, Furyl, Furylmethyl, Thienyl, Thienylmethyl, Pyrrolyl, Pyrrolyl-$C_1$-$C_4$-alkyl, Pyrazolyl, Pyrazolyl-$C_1$-$C_4$-alkyl, Imidazolyl und Imidazolyl-$C_1$-$C_4$-alkyl steht, und
X für Fluor, Chlor, Brom, Amino, Cyanamino oder für einen gegebenenfalls durch Fluor, Chlor, Brom, $C_1$-$C_4$-Alkoxy, $C_1$-$C_4$-Alkylthio, $C_1$-$C_4$-Alkylamino oder Di-($C_1$-$C_4$-alkyl)-amino substituierten Rest aus der Reihe $C_1$-$C_4$-Alkoxy, $C_1$-$C_4$-Alkylthio, $C_1$-$C_4$-Alkylamino und Di-($C_1$-$C_4$-alkyl)-amino steht.

Die Erfindung betrifft insbesondere Verbindungen der Formel (I), in welcher
$R^1$ für gegebenenfalls durch $C_1$-$C_3$-Alkoxy (welches gegebenenfalls durch Fluor substituiert ist) oder durch $C_1$-$C_3$-Alkoxy-$C_1$-$C_3$-alkoxy substituiertes $C_1$-$C_4$-Alkyl steht,
$R^2$ für $C_1$-$C_4$-Alkyl, $C_3$-$C_6$-Cycloalkyl, $C_3$-$C_6$-Cycloalkyl-methyl, für gegebenenfalls durch Fluor, Chlor, Brom, Cyano, Nitro, Methyl, Ethyl, Trifluormethyl, Methoxy und/oder Ethoxy substitutiertes Phenyl oder Naphthyl, für gegebenenfalls durch Fluor, Chlor, Brom, Cyano, Nitro, Methyl, Ethyl, Trifluormethyl, Methoxy und/oder Ethoxy substituiertes Phenyl-$C_1$-$C_3$-alkyl oder Naphthyl-$C_1$-$C_3$-alkyl, für gegebenenfalls durch Fluor, Chlor, Brom, Cyano, Nitro, Methyl, Ethyl, Methoxy und/oder Ethoxy substituiertes Pyridyl oder Pyridylmethyl, oder für gegebenenfalls durch Fluor, Chlor, Brom, Methyl und/oder Ethyl substituiertes Furyl oder Furylmethyl steht, und
X für Chlor, Methoxy, Ethoxy, Methylthio oder Ethylthio steht.

Ganz besonders bevorzugt werden diejenigen Verbindungen der Formel (I), in welcher

$R^1$ für $C_1$-$C_2$-Alkoxy-$C_1$-$C_3$-alkyl steht,
$R^2$ für gegebenenfalls durch Fluor, Chlor, Brom oder Methyl substituiertes (R/S)- oder (S)-1-Phenylethyl steht und
X für Chlor steht.

Verwendet man beispielsweise 4-Chlor-6-isopropylamino-2-methylsulfonyl-pyrimidin und das Kaliumsalz von 3-Methoxy-propanol als Ausgangsstoffe, so kann der Reaktionsablauf beim erfindungsgemäßen Verfahren (a) durch das folgende Formelschema skizziert werden:

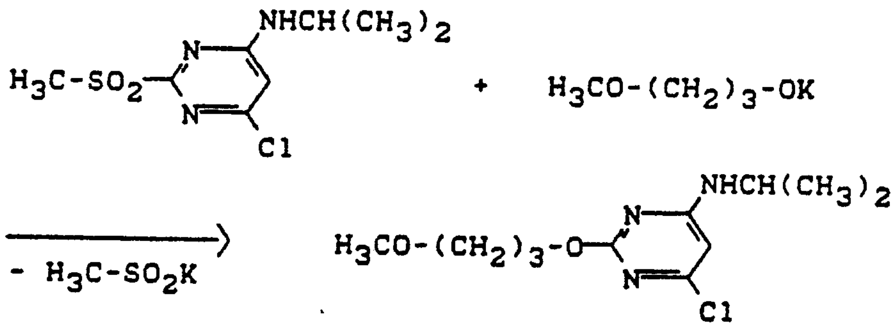


Verwendet man beispielsweise 4-Chlor-6-benzylamino-2-(2-ethoxy-ethoxy)-pyrimidin und Natriumethylat als Ausgangsstoffe, so kann der Reaktionsablauf beim erfindungsgemäßen Verfahren (b) durch das folgende Formelschema skizziert werden:

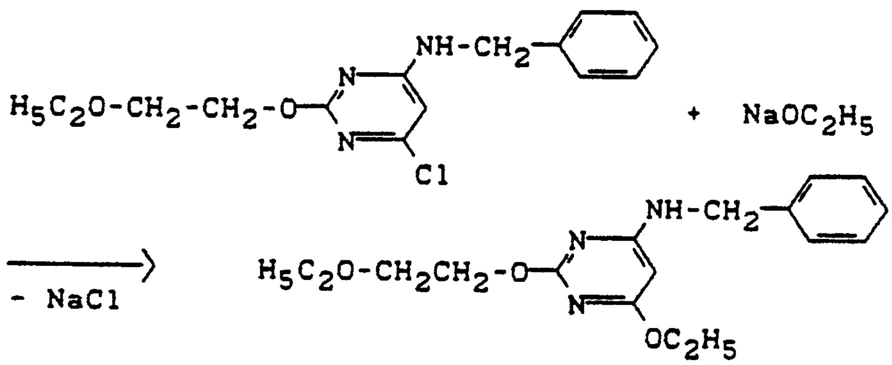


Verwendet man beispielsweise 2-(2-Ethoxy-2-ethoxy-ethoxy)-4,6-dichlor-pyrimidin und 2-(4-Chlor-phenyl)-ethylamin als Ausgangsstoffe, so kann der Reaktionsablauf beim erfindungsgemäßen Verfahren (c) durch das folgende Formelschema skizziert werden:

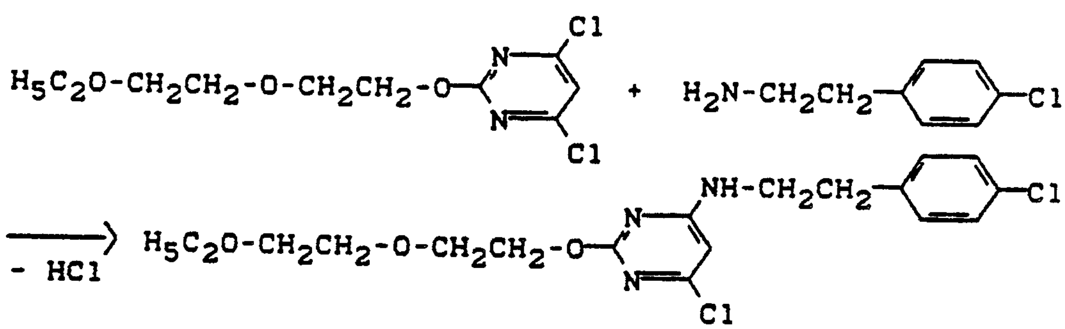


Die beim erfindungsgemäßen Verfahren (a) als Ausgangsstoffe zu verwendenden 2-Alkylsulfonyl-pyrimidin-Derivate sind durch die Formel (II) allgemein definiert. In Formel (II) haben $R^2$ und X vorzugsweise bzw. insbesondere bzw. ganz besonders bevorzugt diejenigen Bedeutungen, die bereits oben im Rahmen der Beschreibung der erfindungsgemäßen Verbindungen der Formel (I) als bevorzugt bzw. als insbesondere bevorzugt bzw. als ganz besonders bevorzugt für $R^2$ und X angegeben wurden und $R^3$ steht vorzugsweise

für $C_1$-$C_4$-Alkyl, insbesondere für Methyl.

Beispiele für die Ausgangsstoffe der Formel (II) sind in der nachstehenden Tabelle 1 aufgeführt.

**Tabelle 1:** Beispiele für die Ausgangsstoffe der Formel (II)

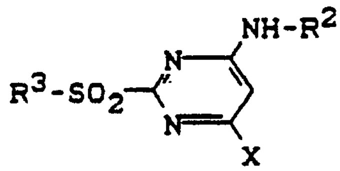

(II)

Tabelle 1

| $R^2$ | $R^3$ | X |
|---|---|---|
| 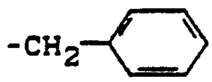 | $CH_3$ | Cl |
| $C_2H_5$ | $CH_3$ | Cl |
| $C_3H_7$ | $CH_3$ | Cl |
| $CH(CH_3)_2$ | $CH_3$ | Cl |
| $C_4H_9$ | $CH_3$ | Cl |
| $CH_2CH(CH_3)_2$ | $CH_3$ | Cl |
| 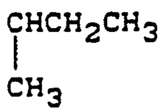 | $CH_3$ | Cl |
| $C(CH_3)_3$ | $CH_3$ | Cl |
| 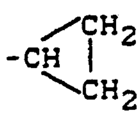 | $CH_3$ | Cl |
| 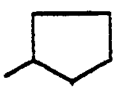 | $CH_3$ | Cl |
| 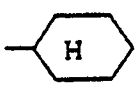 | $CH_3$ | Cl |
| 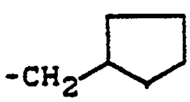 | $CH_3$ | Cl |
| 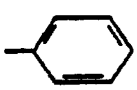 | $CH_3$ | Cl |

Tabelle 1 - Fortsetzung

| $R^2$ | $R^3$ | X |
|---|---|---|
| 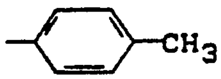 | $CH_3$ | Cl |
| 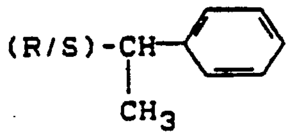 | $CH_3$ | Cl |
| 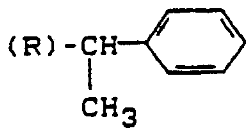 | $CH_3$ | Cl |
| 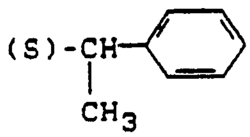 | $CH_3$ | Cl |
| 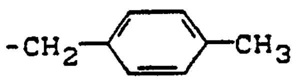 | $CH_3$ | Cl |
| 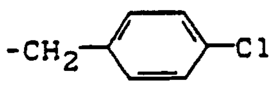 | $CH_3$ | Cl |
| 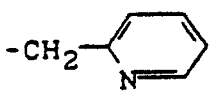 | $CH_3$ | Cl |
| 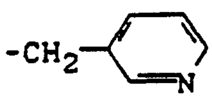 | $CH_3$ | Cl |
| 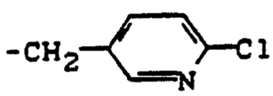 | $CH_3$ | Cl |
| 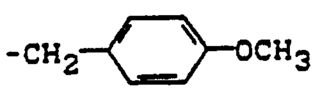 | $CH_3$ | Cl |

<u>Tabelle 1</u> - Fortsetzung

| $R^2$ | $R^3$ | X |
|---|---|---|
| 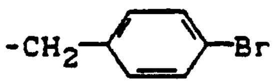 | $CH_3$ | Cl |
| 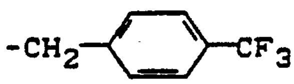 | $CH_3$ | Cl |
| 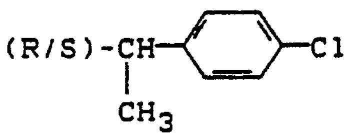 | $CH_3$ | Cl |
| 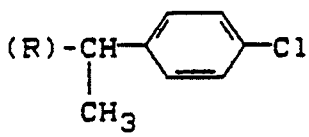 | $CH_3$ | Cl |
| 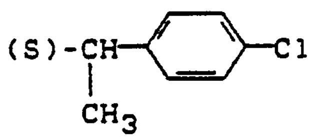 | $CH_3$ | Cl |
| 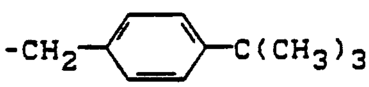 | $CH_3$ | Cl |
| 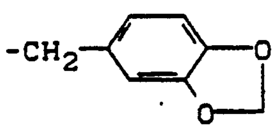 | $CH_3$ | Cl |
| 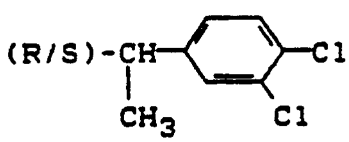 | $CH_3$ | Cl |
| 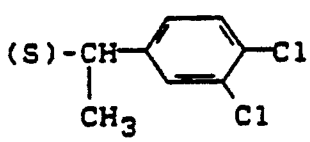 | $CH_3$ | Cl |

<u>Tabelle 1</u> - Fortsetzung

| $R^2$ | $R^3$ | X |
|---|---|---|
| 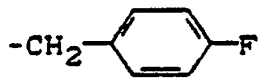 | $CH_3$ | Cl |
| 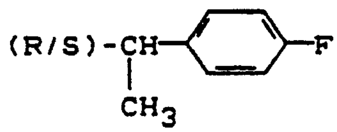 | $CH_3$ | Cl |
| 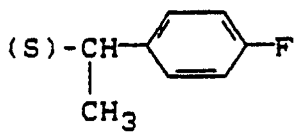 | $CH_3$ | Cl |
| 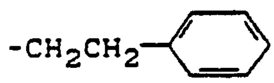 | $CH_3$ | Cl |
| 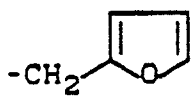 | $CH_3$ | Cl |
| 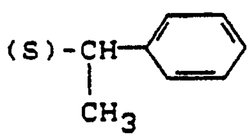 | $CH_3$ | $OCH_3$ |

Die Ausgangsstoffe der Formel (II) sind noch nicht aus der Literatur bekannt. Man erhält die neuen 2-Alkylsulfonyl-pyrimidin-Derivate der allgemeinen Formel (II), wenn man entsprechende 2-Alkylthio-pyrimidin-Derivate der allgemeinen Formel (VII)

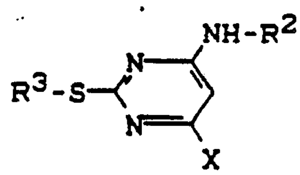 (VII)

in welcher
$R^2$, $R^3$ und X die oben angegebenen Bedeutungen haben,
mit Oxidationsmitteln, wie z.B. Hydrogenperoxid, gegebenenfalls in Gegenwart von Katalysatoren, wie z. B. Ammoniummolybdat und Ameisensäure, und gegebenenfalls in Gegenwart von Verdünnungsmitteln, wie z. B. Methylenchlorid, bei Temperaturen zwischen 0 °C und 50 °C umsetzt und nach üblichen Methoden aufarbeitet.

In Formel (VII) haben $R^2$ und X vorzugsweise bzw. insbesondere bzw. ganz besonders bevorzugt diejenigen Bedeutungen, die bereits oben im Rahmen der Beschreibung der erfindungsgemäßen Verbindungen der Formel (I) als bevorzugt bzw. als insbesondere bevorzugt bzw. als ganz besonders bevorzugt für $R^2$ und X angegeben wurden und $R^3$ steht vorzugsweise für $C_1$-$C_4$-Alkyl, insbesondere für Methyl.

Beispielhafte Bedeutungen für $R^2$, $R^3$ und X wurden bereits in Tabelle 1 angegeben.

Die Zwischenprodukte der Formel (VII) sind noch nicht aus der Literatur bekannt. Man erhält die neuen 2-Alkylthio-pyrimidin-Derivate der allgemeinen Formel (VII), wenn man 2-Alkylthio-4,6-dichlor-pyrimidin-Derivate der allgemeinen Formel (VIII)

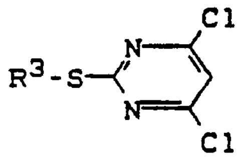

(VIII)

in welcher $R^3$ die oben angegebene Bedeutung hat, mit Aminoverbindungen der allgemeinen Formel (VI)

$H_2N - R^2$ (VI)

in welcher $R^2$ die oben angegebene Bedeutung hat, und gegebenenfalls anschließend mit Verbindungen der allgemeinen Formel (IV)

$M^{\cdot} - X$ (IV)

in welcher $M^{\cdot}$ und X die oben angegebenen Bedeutungen haben, gegebenenfalls in Gegenwart von Säureakzeptoren, wie z. B. Triethylamin, und gegebenenfalls in Gegenwart von Verdünnungsmitteln, wie z. B. Toluol, Tetrahydrofuran oder Dioxan bei Temperaturen zwischen 0 °C und 150 °C umsetzt und nach üblichen Methoden aufarbeitet.

Die Verbindungen der Formel (VIII) sind bekannt und/oder können nach an sich bekannten Verfahren hergestellt werden (vgl. J. Org. Chem. 26 (1961), 792; US-Patent 4 199 583).

Die Verbindungen der Formel (VI) und (IV) sind bekannte Synthesechemikalien.

Die beim erfindungsgemäßen Verfahren weiter als Ausgangsstoffe zu verwendenden Metall-Alkoholate sind durch die Formel (III) allgemein definiert. In Formel (III) hat $R^1$ vorzugsweise bzw. insbesondere bzw. ganz besonders bevorzugt diejenige Bedeutung, die bereits im Rahmen der Beschreibung der erfindungsgemäßen Verbindungen der Formel (I) als bevorzugt bzw. als insbesondere bevorzugt bzw. als ganz besonders bevorzugt angegeben wurde und M steht vorzugsweise für Natrium oder Kalium.

Als Beispiele für die Ausgangsstoffe der Formel (III) seien genannt die Natriumsalze und die Kaliumsalze von 2-Methoxy-ethanol, 2-Ethoxy-ethanol, 3-Methoxy-propanol, 2-Methoxy-propanol, 3-Ethoxy-propanol, 2-(2-Methoxy-ethoxy)-ethanol, 2-(2-Ethoxy-ethoxy)-ethanol und 2-(2,2,2-Trifluorethoxy)-ethanol.

Die Verbindungen der Formel (III) sind bekannt und/oder können auf einfache Weise aus entsprechenden Alkoholen und geeigneten Metallen, wie z. B. Natrium oder Kalium, hergestellt werden.

Die beim erfindungsgemäßen Verfahren (b) als Ausgangsstoffe zu verwendenden 4-Halogen-pyrimidin-Derivate sind durch die Formel (Ia) allgemein definiert. In Formel (Ia) haben $R^1$ und $R^2$ vorzugsweise bzw. insbesondere bzw. ganz besonders bevorzugt diejenigen Bedeutungen, die bereits im Rahmen der Beschreibung der erfindungsgemäßen Verbindungen der Formel (I) als bevorzugt bzw. als insbesondere bevorzugt bzw. als ganz besonders bevorzugt für $R^1$ und $R^2$ angegeben wurden und $X^1$ steht vorzugsweise für Chlor.

Beispiele für die Ausgangsstoffe der Formel (Ia) sind in der nachstehenden Tabelle 2 aufgeführt.

**Tabelle 2: Beispiele für die Ausgangsstoffe der Formel (Ia)**

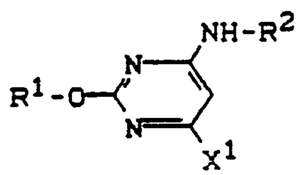

(Ia)

<u>Tabelle 2</u>

| $R^1$ | $R^2$ | $X^1$ |
|---|---|---|
| $-CH_2CH_2-OCH_3$ | $-CH_2$ 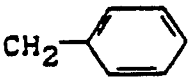 | Cl |
| $-CH_2CH_2-OC_2H_5$ | $-CH_2$ 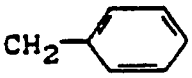 | Cl |
| $-(CH_2)_3-OCH_3$ | $-CH_2$ 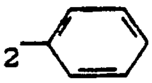 | Cl |
| $-CH_2CH_2-OCH_3$ | $C_2H_5$ | Cl |
| $-CH(CH_3)-CH_2-OCH_3$ | $C_3H_7$ | Cl |
| $-CH_2CH_2-OCH_3$ | $CH(CH_3)_2$ | Cl |
| $-CH_2-OC_2H_5$ | $C_4H_9$ | Cl |
| $-CH_2CH_2-OCH_3$ | 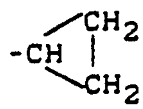 | Cl |
| $-CH_2CH_2-OC_2H_5$ | 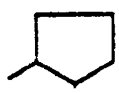 | Cl |
| $-CH_2CH_2-OCH_3$ | $-CH_2$ 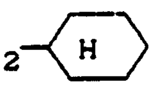 | Cl |
| $-CH_2CH_2-OC_2H_5$ | $CH_2CH(CH_3)_2$ | Cl |
| $-CH_2CH_2-OCH_3$ | (R/S)- 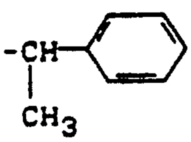 | Cl |

<u>Tabelle 2</u> - Fortsetzung

| $R^1$ | $R^2$ | $X^1$ |
|---|---|---|
| $-CH_2CH_2-OCH_3$ | 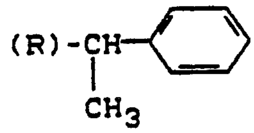 | Cl |
| $-CH_2CH_2-OCH_3$ | 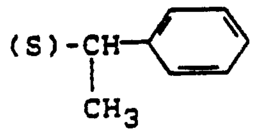 | Cl |
| $-CH_2CH_2-OC_2H_5$ | 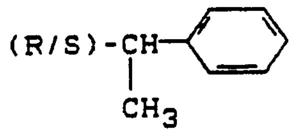 | Cl |
| $-CH_2CH_2-OCH_3$ | 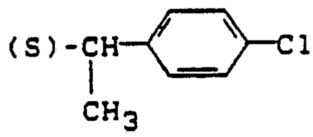 | Cl |
| $-CH_2CH_2-OC_2H_5$ | 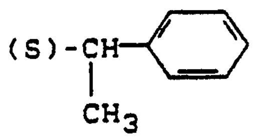 | Cl |
| $-CH_2CH_2-OCH_3$ | 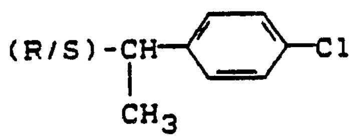 | Cl |
| $-CH_2CH_2-OCH_3$ | 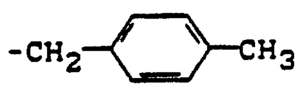 | Cl |
| $-CH_2CH_2-OC_2H_5$ | 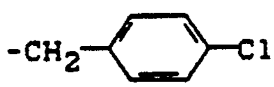 | Cl |
| $-CH_2CH_2-OCH_3$ | 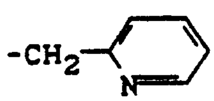 | Cl |

<u>Tabelle 2</u> - Fortsetzung

| $R^1$ | $R^2$ | $X^1$ |
|---|---|---|
| $-CH_2CH_2-OC_2H_5$ | 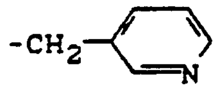 | Cl |
| $-CH_2CH_2-OCH_3$ | 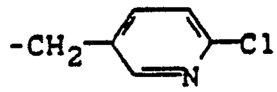 | Cl |
| $-CH_2CH_2-OC_2H_5$ | 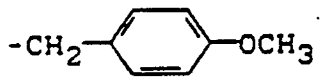 | Cl |
| $-CH_2CH_2-OCH_3$ | 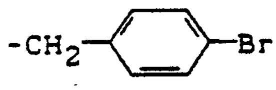 | Cl |
| $-CH_2CH_2-OC_2H_5$ | 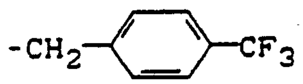 | Cl |
| $-(CH_2)_3-OCH_3$ | 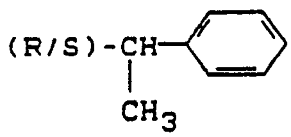 | Cl |
| $-(CH_2)_3-OCH_3$ | 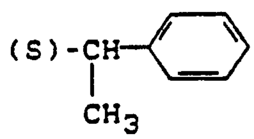 | Cl |
| $-CH_2CH_2-OC_2H_5$ | 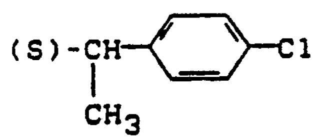 | Cl |
| $-CH_2CH_2-OC_2H_5$ | 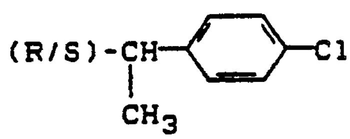 | Cl |
| $-CH_2CH_2-OCH_3$ | 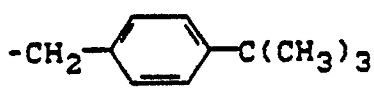 | Cl |

Tabelle 2 - Fortsetzung

| $R^1$ | $R^2$ | $X^1$ |
|---|---|---|
| $-CH_2CH_2-OC_2H_5$ | 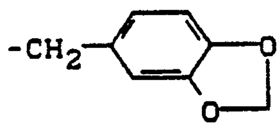 | Cl |
| $-CH_2CH_2-OCH_3$ | 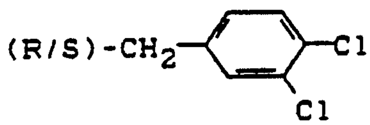 | Cl |
| $-CH_2CH_2-OC_2H_5$ | 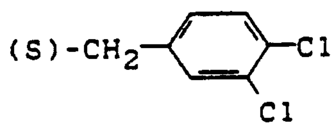 | Cl |
| $-(CH_2)_3-OCH_3$ | 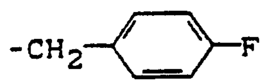 | Cl |
| $-CH_2CH_2-OCH_3$ | 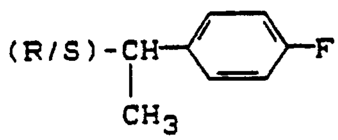 | Cl |
| $-CH_2CH_2-OC_2H_5$ | 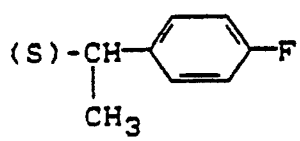 | Cl |
| $-CH_2CH_2-OCH_3$ | 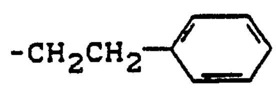 | Cl |
| $-CH_2CH_2-OC_2H_5$ | 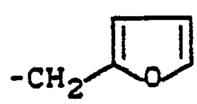 | Cl |
| $-CH_2CH_2-OCH_2-CF_3$ | 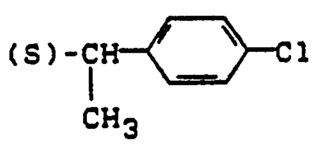 | Cl |

Tabelle 2 - Fortsetzung

| $R^1$ | $R^2$ | $X^1$ |
|---|---|---|
| $-CH_2CH_2-OC_2H_4-OC_2H_5$ | 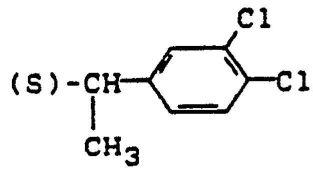 | F |
| $-CH_2CH_2-OC_2H_4-OCH_3$ | 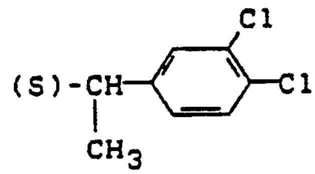 | F |

Die Ausgangsstoffe der Formel (Ia) sind auch erfindungsgemäße Wirkstoffe, welche zum Definitionsbereich der Formel (I) gehören. Sie können nach dem erfindungsgemäßen Verfahren (a) hergestellt werden.

Die bei Verfahren (b) weiter als Ausgangsstoffe einzusetzenden Verbindungen sind durch die Formel (IV) allgemein definiert. In Formel (IV) hat X vorzugsweise bzw. insbesondere bzw. ganz besonders bevorzugt diejenige Bedeutung, die bereits im Rahmen der Beschreibung der erfindungsgemäßen Verbindungen der Formel (I) als bevorzugt bzw. als insbesondere bevorzugt bzw. als ganz besonders bevorzugt für X angegeben wurde und $M^1$ steht vorzugsweise für Wasserstoff, Lithium, Natrium oder Kalium, insbesondere für Natrium oder Kalium.

Als Beispiele für die Ausgangsstoffe der Formel (IV) seien genannt:

Natrium- und Kalium-fluorid, Natrium- und Kalium-bromid, Natrium- und Kalium-iodid, Ammoniak, Natrium- und Kalium-amid, Methanol, Ethanol, Propanol, Isopropanol, Butanol und 2-Methyl-propanol sowie deren Natrium- und Kaliumsalze, Methanthiol, Ethanthiol, Propanthiol, 1-Methyl-ethanthiol, Butanthiol, 1-Methyl- und 2-Methyl-propanthiol sowie deren Natrium- und Kalium-salze, Methylamin, Ethylamin, Propylamin, Isopropylamin, Butylamin, Isobutylamin, Dimethylamin, Diethylamin und Cyanamid.

Die Ausgangsstoffe der Formel (IV) sind bekannte Synthesechemikalien.

Die beim erfindungsgemäßen Verfahren (c) als Ausgangsstoffe zu verwendenden 4-Halogen-pyrimidin-Derivate sind durch die Formel (V) allgemein definiert.

In Formel (V) haben $R^1$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und X angegeben wurden und $X^1$ steht vorzugsweise für Chlor.

Als Beispiele für die Ausgangsstoffe der Formel (V) seien genannt:

2-(2-Methoxy-ethoxy)-, 2-(2-Ethoxy-ethoxy)-, 2-(3-Methoxy-propoxy)-, 2-(3-Ethoxy-propoxy)-, 2-(2-Methoxy-propoxy)- und 2-(2-Ethoxy-propoxy)-4,6-dichlor-pyrimidin.

Die Ausgangsstoffe der Formel (V) sind bekannt und/oder können nach an sich bekannten Methoden hergestellt werden, ausgehend z.B. von 2,4,6-Trichlor-pyrimidin oder 2-Methylsulfonyl-4,6-dichlor-pyrimidin (vgl. z.B. Liebig's Ann. Chem. 1975, S. 1113 ff. und Austral. J. Chem. Bd. 18, S. 199 ff).

Die beim erfindungsgemäßen Verfahren (c) weiter als Ausgangsstoffe zu verwendenden Aminoverbindungen sind durch die Formel (VI) allgemein definiert.

In Formel (VI) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$ angegeben wurde.

Als Beispiele für die Ausgangsstoffe der Formel (VI) seien genannt:

Methylamin, Ethylamin, Propylamin, Isopropylamin, Butylamin, Isobutylamin, Cyclopropylamin, Cyclobutylamin, Cyclopentylamin, Cyclohexylamin, Cyclopentylmethylamin, Cyclohexylmethylamin, Benzylamin, 1-Phenyl-ethylamin, 1-(4-Fluor-phenyl)-ethylamin, 1-(4-Chlor-phenyl)-ethylamin, 1-(4-Brom-phenyl)-ethylamin, 1-(4-Methyl-phenyl)-ethylamin, 1-(4-Trifluormethyl-phenyl)-ethylamin, 1-(4-Methoxy-phenyl)-ethylamin und 1-(3,4-Dichlor-phenyl)-ethylamin.

Die Ausgangsstoffe der Formel (VI) sind bekannte Synthesechemikalien.

Das erfindungsgemäße Verfahren (a) wird vorzugsweise in Gegenwart eines Verdünnungsmittels durchgeführt. Als solche kommen praktisch alle inerten organischen Lösungsmittel, insbesondere jedoch aprotisch polare Solventien in Betracht. Hierzu gehören chlorierte Kohlenwasserstoffe wie z. B. Methylenchlorid, Chloroform oder Chlorbenzol, Ketone wie z. B. Aceton, Methylethylketon, Methylisopropylketon und Methylisobutylketon, Nitrile wie z. B. Acetonitril und Propionitril, Ether wie z. B. Diethylether, Diisopropylether, Dimethoxyethan, Tetrahydrofuran und Dioxan, Amide wie z. B. Dimethylformamid und Dimethylacetamid sowie Dimethylsulfoxid und Sulfolan.

Aus der genannten Gruppe von aprotisch polaren Lösungsmitteln sind die folgenden besonders bevorzugt:
Dimethylformamid, Dimethylacetamid, Acetonitril und Propionitril.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C. Verfahren (a) wird im allgemeinen bei Normaldruck durchgeführt.

Zur Durchführung des erfindungsgemäßen Verfahrens (a) setzt man je Mol 2-Alkylsulfonyl-pyrimidin-Derivat der Formel (II) im allgemeinen zwischen 1 und 10 Mol, vorzugsweise zwischen 1 und 5 Mol, Metall-Alkoholat der Formel (III) ein. Die Reaktionskomponenten werden gewöhnlich bei Raumtemperatur oder unter leichtem Kühlen vermischt und bis zum Reaktionsende gerührt. Die Aufarbeitung kann nach üblichen Methoden durchgeführt werden. Beispielsweise wird - gegebenenfalls nach Einengen - mit Wasser und einem mit Wasser praktisch nicht mischbaren organischen Lösungsmittel, wie z. B. Methylenchlorid, geschüttelt, die organische Phase gegebenenfalls getrocknet und filtriert. Vom Filtrat wird das Lösungsmittel unter vermindertem Druck sorgfältig abdestilliert. Der verbleibende Rückstand enthält im wesentlichen das Produkt der Formel (I).

Das erfindungsgemäße Verfahren (b) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8- Diazabicyclo-[5,4,0,]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man je Mol 4-Halogen-pyrimidin-Derivat der Formel (Ia) im allgemeinen zwischen 1 und 10 Mol, vorzugsweise zwischen 1 und 5 Mol, Verbindung der Formel (IV) ein. Die Umsetzung und die Aufarbeitung können wie für Verfahren (a) angegeben durchgeführt werden.

Das erfindungsgemäße Verfahren (c) wird vorzugsweise unter Verwendung von Verdünnungsmitteln durchgeführt. Als solche kommen insbesondere diejenigen organischen Lösungsmittel in Betracht, die oben im Zusammenhang mit der Beschreibung des erfindungsgemäßen Verfahrens (b) genannt werden.

Als Säureakzeptoren können bei dem erfindungsgemäßen Verfahren (c) alle üblicherweise für derartige Umsetzungen verwendbaren Säurebindemittel eingesetzt werden. Vorzugsweise kommen diejenigen Säureakzeptoren in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man je Mol 4-Halogen-pyrimidin-Derivat der Formel (V) im allgemeinen zwischen 1 und 3 Mol, vorzugsweise zwischen 1 und 2 Mol, Aminoverbindung der Formel (VI) ein. Die Umsetzung und die Aufarbeitung können wie für Verfahren (a) angegeben durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf In dustrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung monokotyler und dikotyler Unkräuter, insbesondere in monokotylen Kulturen, vorzugsweise im Nachauflauf-Verfahren.

Einige der erfindungsgemäßen Verbindungen zeigen auch fungizide Wirkung, beispielsweise gegen echten Mehltau an Gurken oder gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol

oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate, sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetate, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkraut bekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylithio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TER BUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 3,5,6-Trichlor-2-pyridyloxyessigsäure (TRICLOPYR). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15

kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

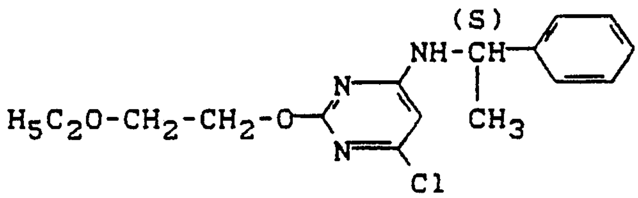

(Verfahren (a))

Eine Mischung aus 3,3 g (0,011 Mol) (S)-4-Chlor-2-methylsulfonyl-6-(1-phenyl-ethylamino)-pyrimidin, 4,0 g (0,036 Mol) 2-Ethoxy-ethanol-Natriumsalz und 50 ml Acetonitril wird 12 Stunden bei 20 °C gerührt. Nach Einengen wird mit Methylenchlorid/Wasser geschüttelt, die organische Phase mit Natriumsulfat getrocknet und filtriert. Vom Filtrat wird das Lösungsmittel im Wasserstrahlvakuum sorgfältig abdestilliert.

Man erhält 2,9 g (85 % der Theorie) (S)-4-Chlor-2-(2-ethoxy-ethoxy)-6-(1-phenyl-ethylamino)-pyrimidin als hellbraunen öligen Rückstand.

$^1$H-NMR ($CDCl_3$, 300 MHz), $\delta$ = 1,20 (t, $CH_2\underline{CH_3}$); 1,55 (d, $CH\underline{CH_3}$); 3,55 (q, $\underline{CH_2}CH_3$); 3,71 und 4,38 (m, $\underline{OCH_2CH_2O}$).

Analog Beispiel 1 bzw. entsprechend der allgemeinen Beschreibung der erfindungsgemäßen Herstellungsverfahren können die in die nachstehenden Tabelle 3 aufgeführten Verbindungen der Formel (I) hergestellt werden.

<u>Tabelle 3:</u> Beispiele für die Verbindungen der Formel (I)

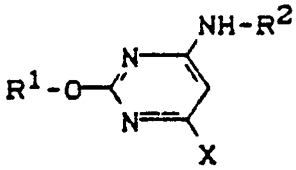

(I)

| Bsp.-Nr. | $R^1$ | $R^2$ | X | Physikal. Konstante |
|---|---|---|---|---|
| 2 | $-CH_2CH_2-OC_2H_5$ | 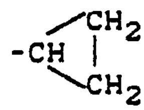 | Cl | 76 °C |
| 3 | $-CH_2CH_2-OC_2H_5$ | 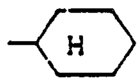 | Cl | NMR-Spektrum* |
| 4 | $-CH_2CH_2-OCH_3$ | 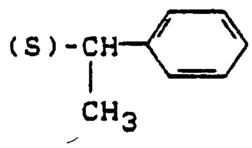 | Cl | $n_D^{22}=1.5680$ |
| 5 | $-CH_2CH_2-OCH_3$ | 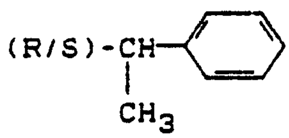 | Cl | |
| 6 | $-CH_2CH_2-OC_2H_5$ | 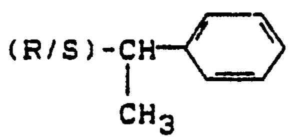 | Cl | |
| 7 | $-(CH_2)_3-OCH_3$ | 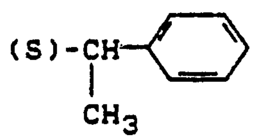 | Cl | |

* $^1$H-NMR zu Beispiel 3 ($CDCl_3$, 300 MHz), $\delta$ = 1,21 (t, $CH_3$); 1,13- 2,05 (m, $C_6H_{11}$), 3,57 (q, $OC\underline{H}_2CH_3$); 3,76 (m, $OCH_2$); 4,41 (m, $OCH_2$); 5,06 (s, NH); 5,98 (s, CH).

Tabelle 3 - Fortsetzung

| Bsp.-Nr. | $R^1$ | $R^2$ | X | Physikal. Konstante |
|---|---|---|---|---|
| 8 | $-C_2H_4-O-C_2H_4-OCH_3$ | 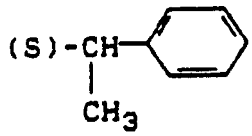 | Cl | $n_D^{22}=1.5582$ |
| 9 | $-C_2H_4-O-C_2H_4-OC_2H_5$ | 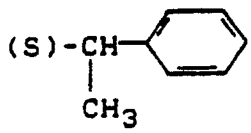 | Cl | $n_D^{21}=1.5419$ |
| 10 | $-C_2H_4-OCH_2-CF_3$ | 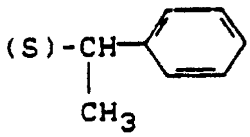 | Cl | $n_D^{22}=1.4999$ |
| 11 | $-CH_2CH_2-OCH_3$ | 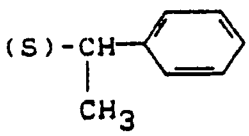 | $OCH_3$ | $n_D^{23}=1.5604$ |
| 12 | $-CH_2CH_2-O-C_2H_4-OCH_3$ | 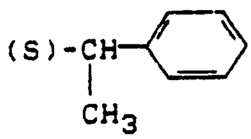 | $OCH_3$ | $n_D^{23}=1.5456$ |

Ausgangsstoffe der Formel (II)

Beispiel (II-1)

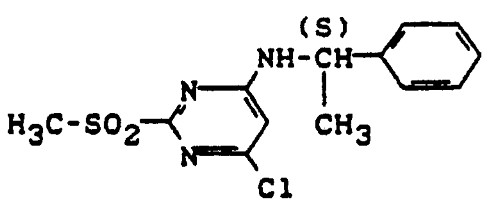

5,4 g (0,019 Mol) (S)-4-Chlor-2-methylthio-6-(1-phenyl-ethylamino)-pyrimidin werden in 100 ml Methylenchlorid gelöst. Nach Zugabe von 2 g Ameisensäure und 0,1 g Ammoniummolybdat werden unter Rühren 6,0 g 35 %ige wäßrige Hydrogenperoxidlösung tropfenweise zur Reaktionsmischung gegeben. Nach 12stündigem Rühren bei 20 °C wird die organische Phase abgetrennt, mit stark verdünnter Natriumhydrogensulfit-Lösung gewaschen, mit Natriumsulfat getrocknet und filtiert. Vom Filtrat wird das Lösungsmittel im Wasserstrahlvakuum sorgfältig abdestilliert.

Man erhält 5,2 g (86 % der Theorie) (S)-(4-Chlor-2-methylsulfonyl-6-(1-phenyl-ethylamino)-pyrimidin als gelblichen öligen Rückstand.

$^{13}$C-NMR ($CDCl_3$;22,6 MHz), $\delta$ = 22,3 ($\underline{C}H_3$), 30,7 ($SO_2\underline{C}H_3$), 51,9($\underline{C}HCH_3$).

Ausgangsstoffe der Formel (VII)

Beispiel (VII-1)

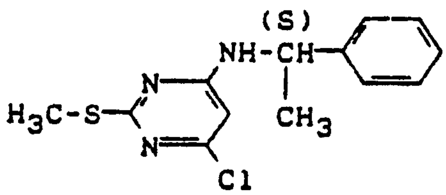

Eine Mischung aus 5,9 (0,03 Mol) 4,6-Dichlor-2-methylthio-pyrimidin, 3,1 g (0,031 Mol) Triethylamin, 2,7 g (0,031 Mol) (S)-1-Phenyl-ethylamin und 80 ml Toluol wird 12 Stunden unter Rückfluß zum Sieden erhitzt. Nach Einengen wird der Rückstand in Methylenchlorid aufgenommen, mit Wasser gewaschen, mit Natriumsulfat getrocknet und filtriert. Vom Filtrat wird das Lösungsmittel im Wasserstrahlvakuum sorgfältig abdestilliert.

Man erhält 7,0 g (83 % Theorie) (S)-4-Chlor-2-methylthio-6-(1-phenyl-ethylamino)-pyrimidin als gelblichen öligen Rückstand; NMR-Spektrum;
$^1$H-NMR ($CDCl_3$, 300 MHz), $\delta$ = 1,56 (d, $CHC\underline{H}_3$); 2,45 (s, $SC\underline{H}_3$).

Anwendungsbeispiel

Im folgenden Anwendungsbeispiel wird die Verbindung der nachstehenden Formel als Vergleichssubstanz eingesetzt;

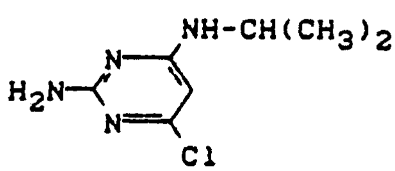

(A)

2-Amino-4-chlor-6-isopropylamino-pyrimidin
(bekannt aus DE-OS 20 06 145).

Beispiel A

Post-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit gegenüber dem Stand der Technik zeigt in diesem Test z. B. die Verbindung gemäß Herstellungsbeispiel: (1).

## Ansprüche

1. Pyrimidin-Derivate der allgemeinen Formel (I)

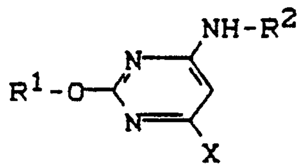

(I)

in welcher
$R^1$ für gegebenenfalls substituiertes Alkyl steht,
$R^2$ für einen gegebenenfalls substituierten Rest aus der Reihe Alkyl, Cycloalkyl, Cycloalkylalkyl, Aryl, Aralkyl, Heteroaryl und Heteroarylalkyl steht, und
X für Halogen, Amino, Cyanamino oder für einen gegebenenfalls substituierten Rest aus der Reihe Alkoxy, Alkylthio, Alkylamino und Dialkylamino steht.

2. Pyrimidin-Derivate der allgemeinen Formel (I) gemäß Anspruch 1, dadurch gekennzeichnet, daß darin
$R^1$ für gegebenenfalls durch Fluor, Chlor, Brom, $C_1$-$C_4$-Alkoxy (welches gegebenenfalls durch Fluor und/oder Chlor substituiert ist), $C_1$-$C_4$-Alkoxy-$C_1$-$C_4$-alkoxy, $C_1$-$C_4$-Alkylthio, $C_1$-$C_4$-Alkylamino oder Di-($C_1$-$C_4$-alkyl)-amino substituiertes $C_1$-$C_6$-Alkyl steht,
$R^2$ für gegebenenfalls durch Fluor, Chlor, $C_1$-$C_4$-Alkoxy, $C_1$-$C_4$-Alkylthio, $C_1$-$C_4$-Alkylamino oder Di-($C_1$-$C_4$-alkyl)-amino substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl oder $C_3$-$C_6$-Cycloalkyl-$C_1$-$C_3$alkyl, oder für eine gegebenenfalls durch Fluor, Chlor, Brom, Iod, Cyano, Nitro, $C_1$-$C_4$-Alkyl (welches gegebenenfalls durch Fluor und/oder Chlor substituiert ist), $C_1$-$C_4$-Alkoxy oder $C_1$-$C_2$-Alkylendioxy (welche gegebenenfalls durch Fluor und/oder Chlor substituiert sind), $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), Di-($C_1$-$C_2$-alkyl)-amino und/oder durch $C_1$-$C_4$-Alkoxy-carbonyl substituierte Gruppierung aus der Reihe Phenyl, Naphthyl, Phenyl-$C_1$-$C_4$-alkyl, Naphthyl-$C_1$-$C_4$-alkyl, Pyridyl, Pyridyl-$C_1$-$C_4$-alkyl, Chinolinyl, Chinolinyl-$C_1$-$C_4$-alkyl, Isochinolinyl, Isochinolinyl-$C_1$-$C_4$-alkyl, Pyrimidinyl, Pyrimidinyl-$C_1$-$C_4$-alkyl, Furyl, Furylmethyl, Thienyl, Thienylmethyl, Pyrrolyl, Pyrrolyl-$C_1$-$C_4$-alkyl, Pyrazolyl, Pyrazolyl-$C_1$-$C_4$-alkyl, Imidazolyl und Imidazolyl-$C_1$-$C_4$-alkyl steht, und
X für Fluor, Chlor, Brom, Amino, Cyanamino oder für einen gegebenenfalls durch Fluor, Chlor, Brom, $C_1$-$C_4$-Alkoxy, $C_1$-$C_4$-Alkylthio, $C_1$-$C_4$-Alkylamino oder Di-($C_1$-$C_4$-alkyl)-amino substituierten Rest aus der Reihe $C_1$-$C_4$-Alkoxy, $C_1$-$C_4$-Alkylthio, $C_1$-$C_4$-Alkylamino und Di-($C_1$-$C_4$-alkyl)-amino steht.

3. Pyrimidin-Derivate der allgemeinen Formel (I) gemäß Anspruch 1, dadurch gekennzeichnet, daß darin
$R^1$ für gegebenenfalls durch $C_1$-$C_3$-Alkoxy (welches gegebenenfalls durch Fluor substituiert ist) oder durch $C_1$-$C_3$-Alkoxy-$C_1$-$C_3$-alkoxy substituiertes $C_1$-$C_4$-Alkyl steht,
$R^2$ für $C_1$-$C_4$-Alkyl, $C_3$-$C_6$-Cycloalkyl, $C_3$-$C_6$-Cycloalkyl-methyl, für gegebenenfalls durch Fluor, Chlor, Brom, Cyano, Nitro, Methyl, Ethyl, Trifluormethyl, Methoxy und/oder Ethoxy substitutiertes Phenyl oder Naphthyl, für gegebenenfalls durch Fluor, Chlor, Brom, Cyano, Nitro, Methyl, Ethyl, Trifluormethyl, Methoxy und/oder Ethoxy substituiertes Phenyl- $C_1$-$C_3$-alkyl oder Naphthyl-$C_1$-$C_3$-alkyl, für gegebenenfalls durch Fluor, Chlor, Brom, Cyano, Nitro, Methyl, Ethyl, Methoxy und/oder Ethoxy substituiertes Pyridyl oder Pyridylmethyl, oder für gegebenenfalls durch Fluor, Chlor, Brom, Methyl und/oder Ethyl substituiertes Furyl oder Furylmethyl steht, und
X für Chlor, Methoxy, Ethoxy, Methylthio oder Ethylthio steht.

4. Pyrimidin-Derivate der allgemeinen Formel (I) gemäß Anspruch 1, dadurch gekennzeichnet, daß darin $R^1$ für $C_1$-$C_2$-Alkoxy-$C_1$-$C_3$-alkyl steht, $R^2$ für gegebenenfalls durch Fluor, Chlor, Brom oder Methyl substituiertes (R/S)- oder (S)-1-Phenyl-ethyl steht und X für Chlor steht.

5. Verfahren zur Herstellung von Pyrimidin-Derivaten der allgemeinen Formel (I) gemäß Anspruch 1, dadurch gekennzeichnet, daß man

(a) 2-Alkylsulfonyl-pyrimidin-Derivate der allgemeinen Formel (II)

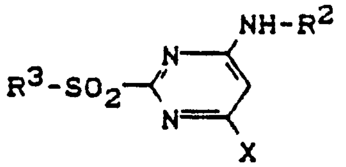

(II)

in welcher
$R^2$ und X die in Anspruch 1 angegebenen Bedeutungen haben und
$R^3$ für gegebenenfalls substituiertes Alkyl oder Benzyl steht,
mit Metall-Alkoholaten der allgemeinen Formel (III)
$R^1$ - O - M (III)
in welcher
$R^1$ die in Anspruch 1 angegebene Bedeutung hat und
M für ein Metalläquivalent steht,
gegebenenfalls in Gegenwart eines Verdünnungsmittels umsetzt, oder daß man

(b) 4-Halogen-pyrimidin-Derivate der allgemeinen Formel (Ia)

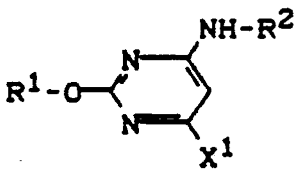

(Ia)

in welcher
$R^1$ und $R^2$ die in Anspruch 1 angegebenen Bedeutungen haben und
$X^1$ für Halogen steht,
mit Verbindungen der allgemeinen Formel (IV)
$M^1$ - X (IV)
in welcher
X die in Anspruch 1 angegebene Bedeutung hat und
$M^1$ für Wasserstoff oder ein Metalläquivalent steht,
gegebenenfalls in Gegenwart eines Säureakzeptors und gegebenenfalls in Gegenwart eines Verdünnungsmittels umsetzt, oder daß man

(c) 4-Halogen-pyrimidin-Derivate der allgemeinen Formel (V)

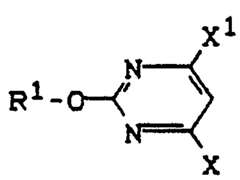

(V)

in welcher
$R^1$, X und $X^1$ die in Anspruch 1 angegebenen Bedeutungen haben,
mit Aminoverbindungen der allgemeinen Formel (VI)
$H_2N$ - $R^2$ (VI)
in welcher

$R^2$ die in Anspruch 1 angegebene Bedeutung hat,
gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einem Pyrimidin-Derivat der Formel (I) gemäß Anspruch 1.

7. Verfahren zur Bekämpfung von Unkräutern, dadurch gekennzeichnet, daß man Pyrimidin-Derivate der Formel (I) gemäß Anspruch 1 auf die Unkräuter und/oder ihren Lebensraum einwirken läßt.

8. Verwendung von Pyrimidin-Derivaten der Formel (I) gemäß Anspruch 1 zur Bekämpfung von Unkräutern.

9. Verfahren zur Herstellung von herbiziden Mitteln, dadurch gekennzeichnet, daß man Pyrimidin-Derivate der Formel (I) gemäß Anspruch 1 mit Streckmitteln und/oder oberflächenaktiven Mitteln vermischt.

European Patent Office

# EUROPEAN SEARCH REPORT

Application number

EP 89103864.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 139 613 (CIBA-GEIGY)<br>* Anspruch 1; Tabellen 1,5, 7 *<br>-- | 1 | C 07 D 239/47<br>A 01 N 43/54 |
| D,A | DE - A - 2 006 145 (SANDOZ)<br>* Ansprüche 1,17; Seite 1, Zeile 11 - Seite 3, Zeile 1; Seite 4, Zeile 27 - Seite 5, Zeile 3 *<br>-- | 1,5-9 | |
| A | CHEMICAL ABSTRACTS, vol. 74, no. 11, March 15, 1971, Columbus, Ohio, USA<br>WENTRUP, CURT "Hetarylnitrenes II"<br>page 361, abstract-no. 53 726a<br>& Tetrahedron 1970, 26(21), 4969-83<br>-- | 1 | TECHNICAL FIELDS SEARCHED (Int. Cl.4)<br>C 07 D 239/00 |
| A | CHEMICAL ABSTRACTS, vol. 100, no. 17, April 23, 1984, Columbus, Ohio, USA<br>HURST, DEREK T. "Synthesis and characterization of some 5,5 -dimethyl-N-pyrimidinylsulfinides"<br>pages 646,647, abstract-no. 139 057a<br>& Aust. J. Chem. 1983, 36(10), 2119-22<br>---- | 1 | |

The present search report has been drawn up for all claims

| Place of search | Date of completion of the search | Examiner |
|---|---|---|
| VIENNA | 07-06-1989 | LUX |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82